# EUROPÄISCHE PATENTSCHRIFT

(11) **EP 2 998 385 B1**
(45) Veröffentlichungstag und Bekanntmachung des Hinweises auf die Patenterteilung: **26.04.2017**
(21) Anmeldenummer: 15184623.5
(22) Anmeldetag: 10.09.2015
(51) Int. Cl.: C12M 1/00, C25B 1/04, C12M 1/107

(54) **VERFAHREN ZUR ERZEUGUNG VON WASSERSTOFF**
METHOD FOR PRODUCING HYDROGEN
PROCEDE DE PRODUCTION D'HYDROGENE

(30) Priorität: 17.09.2014 DE 102014113388
(43) Veröffentlichungstag der Anmeldung: 23.03.2016
(73) Patentinhaber: HyEnTec GmbH, 73119 Zell unter Aichelberg (DE)
(72) Erfinder: Wider, Sebastian, 70193 Stuttgart (DE); Schürstedt, Peter, 73119 Zell unter Aichelberg (DE)
(74) Vertreter: Ruckh, Rainer Gerhard

(56) Entgegenhaltungen:
- EP-A1- 2 438 980
- CN-A- 103 075 305
- DE-A1-102009 018 126
- DE-A1-102012 005 023
- US-A1- 2008 127 646

## Beschreibung

Die Erfindung betrifft ein Verfahren zur Herstellung von Wasserstoff.

Wasserstoff als Energieträger für elektrische Stromquellen, wie insbesondere Brennstoffzellen, hat in jüngerer Vergangenheit eine rasch ansteigende Bedeutung erlangt. So werden Brennstoffzellen bereits in signifikanter Anzahl in Fahrzeugen mit Elektroantrieben und auch in anderen Systemen, wie Heizungssystemen in Gebäuden, eingesetzt.

Derartige Systeme arbeiten erheblich umweltfreundlicher als mit fossilen Energieträgern betriebene Systeme, wie zum Beispiel Kraftfahrzeuge mit Verbrennungsmotoren.

Ein Problem besteht jedoch in einer umweltverträglichen Herstellung und Bereitstellung des Wasserstoffs selbst. Bekannte, industriell erprobte Herstellungsverfahren zur Herstellung von Wasserstoff arbeiten selbst mit fossilen Energieträgern. Ein Beispiel hierfür ist die Herstellung von Wasserstoff nach einem Dampfreformierungsverfahren aus Erdgas.

Prinzipiell könnten regenerative Energiequellen wie Windkraftanlagen oder Photovoltaikanlagen zur Stromerzeugung genutzt werden, wobei dieser Strom dann zur Herstellung von Wasserstoff genutzt wird.

Ein generelles Problem hierbei besteht jedoch darin, dass derartige Anlagen prinzipiell nicht geeignet sind kontinuierlich Strom zu erzeugen. So sind Windkraftanlagen auf einen hinreichend starken Wind angewiesen, während Photovoltaikanlagen bei starker Bewölkung unzureichend und in der Nacht überhaupt keinen Strom produzieren können.

Da der mit derartigen Anlagen hergestellte Strom nicht kontinuierlich zur Verfügung steht, müssten Herstellungsverfahren zur Herstellung des Wasserstoffs eingesetzt werden, die dynamisch arbeiten, wie zum Beispiel PEM (protone exchange membrane) Elektrolyseverfahren. Diese Verfahren sind jedoch sehr aufwändig und teuer, was zu einer entsprechenden Verteuerung des so hergestellten Wasserstoffs führt. Weiterhin nachteilig ist die geringe Lebensdauer der für diese Verfahren eingesetzten Komponenten.

Die DE 10 2009 018 126 A1 betrifft ein Energieversorgungssystem umfassend eine Stromerzeugungseinrichtung zur regenerativen Erzeugung von in ein Stromversorgungsnetz einspeisbarer elektrischer Energie, eine Wasserstofferzeugungseinrichtung zur Wasserstofferzeugung unter Verwendung von elektrischer Energie der regenerativen Stromerzeugungseinrichtung, eine Methanisierungseinrichtung zur Umwandlung von durch die Wasserstofferzeugungseinrichtung erzeugtem Wasserstoff und eines zugeführten Kohlenoxidgases in ein methanhaltiges, in ein Gasversorgungsnetz einspeisbares Gas und vorzugsweise auch eine Verstromungseinrichtung zur Erzeugung elektrischer Energie unter Verwendung von Gas aus dem Gasversorgungsnetz. Die Abnahmeleistung der Wasserstofferzeugungseinrichtung und/oder die Einspeiseleistung der Verstromungseinrichtung kann in Abhängigkeit von einem zeitabhängigen Leistungsbedarf des Stromversorgungsnetzes variabel eingestellt werden.

Die CN 103 075 305 A betrifft ein natürliche Energiequellen nutzendes Energieversorgungssystem mit windgetriebenen Generatoren, einem Wasserstoff-Generator, einem Wasserstoff-Speicher und einer Wasserstoff-Brennstoffzelle. Die erzeugte Energie kann Verbrauchern zur Verfügung gestellt werden oder zur Erzeugung von Wasserstoff genutzt werden. Kann der Energiebedarf nicht mit den Windkraftgeneratoren gedeckt werden, wird Verbrauchern Energie aus der Brennstoffzelle zur Verfügung gestellt.

Die EP 2 438 980 A1 betrifft ein Verfahren zum Bereitstellen und Einsetzen einer methanolhaltigen Flüssigkeit mit den folgenden Schritten:
- Bereitstellen eines Kohlenstoffdioxidgases als Kohlenstofflieferant,
- Bereitstelen eines Wasserstoffgases,
- Bereitstellen eines gasförmigen Ausgangsstoffes, der das Kohlenstoffdioxidgas und Wasserstoffgas umfasst,
- Einbringen des Ausgangsstoffes in einen Reaktor,
- Durchlaufen des Ausgangsstoffes durch eine Reaktionsstrecke des Reaktors, die mindestens teilweise mit einem Katalysator bestückt ist, um auf katalysatorsynthetischem Weg die methanolhaltige Flüssigkeit zu synthetisieren,
- Bereitstellen der methanolhaltigen Flüssigkeit an einem ausgangsseitigen Ende des Reaktors, wobei es sich bei der methanolhaltigen Flüssigkeit um ein Gemisch aus Methanol und Wasser handelt,
- Einsetzen der methanolhaltigen Flüssigkeit in einem Denitrifizierungsprozesses, um einen Stickstoffanteil von Wasser, vorzugsweise aus Abwasser, zu entfernen.

Die US 2008/0127646 A1 betrifft ein System zur Erzeugung von Energie mittels erneuerbarer Energiequellen. Die erzeugte Energie kann zur Erzeugung von Wasserstoff genutzt werden. Das System wird mit einem Systemcontroller so gesteuert, dass die Energiequellen einen maximalen Energie-Output liefern.

Die DE 10 2012 005 023 A1 betrifft eine Anordnung zur autonomen Bereitstellung von Elektrizität über Wasserstoff (Notstromversorgung) umfassend
- Bereitstellen eines elektrischen Stroms aus einer regenerativen Quelle
- Herstellen von Wasserstoff aus Wasser in mindestens einem Elektrolyseur unter Verwendung des elektrischen Stroms aus der mindestens einen erneuerbaren Energiequelle,
- Überführen des gebildeten Wasserstoffes aus dem mindestens einen Elektrolyseur in einen ersten chemischen Reaktor enthaltend mindestens ein Substrat mit einem ausgedehnten π-konjugierten System und mindestens teilweise Hydrierung des Substrates
- Überführen des zumindest teilweise hydrierten Substrates aus dem ersten chemischen Reaktor in mindestens einen Speichertank und ggf. Speicherung des zumindest teilweise hydrierten Substrates in dem Speichertank,
- Überführen des zumindest teilweise hydrierten Substrates aus dem Speichertank in mindestens einen zweiten chemischen Reaktor und Dehydrierung des zumindest teilweise hydrierten Substrates in dem zweiten chemischen Reaktor unter Freisetzung von Wasserstoff, und
- Überführen des Wasserstoffs aus dem zweiten chemischen Reaktor in mindestens eine Anlage oder Maschine zur Wandlung des Wasserstoffs in elektrische Energie
- Abgabe des elektrischen Stroms an den Ort des Bedarfs

Der Erfindung liegt die Aufgabe zugrunde ein Verfahren bereitzustellen, das eine umweltfreundliche und gleichzeitig wirtschaftliche Herstellung von Wasserstoff ermöglicht.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Gemäß dem erfindungsgemäßen Verfahren erfolgt eine Erzeugung von elektrischem Strom aus einer Kombination eines Verbrennungsprozesses in Form einer Verbrennung von regenerativen Energieträgern und eines Photovoltaikprozesses. Der so erzeugte elektrische Strom wird dann für einen kontinuierlichen Elektrolyseprozess zur Erzeugung des Wasserstoffs genutzt. Mit einer Steuereinheit wird der Zeitablauf der Erzeugung von Strom vollständig gesteuert, wobei durch die Steuerung vorgegeben wird, zu welchen Zeiten und in welchem Umfang einerseits der Verbrennungsprozess und andererseits der Photovoltaikprozess zur Erzeugung des für den Elektrolyseprozess erforderlichen Stroms herangezogen wird.

Der Grundgedanke der Erfindung besteht somit darin, zur Erzeugung des für die Herstellung erforderlichen elektrischen Stroms nicht nur eine, sondern zwei verschiedene regenerative Energiequellen beziehungsweise Energieträgern einzusetzen.

Damit wird ein diversitäres Stromerzeugungssystem geschaffen, mittels dessen kontinuierlich, das heißt ohne Ausfallzeiten elektrischer Strom in ausreichendem Maß für einen Elektrolyseprozess zur Herstellung des Wasserstoffs bereitgestellt wird. Der Elektrolyseprozess läuft erfindungsgemäß ebenfalls kontinuierlich ab. Dies hat einerseits den Vorteil, dass damit eine fortlaufende Erzeugung von Wasserstoff ohne Ausfallzeiten ermöglicht wird. Wesentlich hierbei ist, dass zur Erzeugung des elektrischen Stroms nur regenerative Energiequellen eingesetzt werden, so dass eine umweltfreundliche Herstellung von Wasserstoff ermöglicht wird.

Weiterhin ist vorteilhaft, dass auf wirtschaftliche Elektrolyseprozesse zurückgegriffen werden kann, wie zum Beispiel eine alkalische Elektrolyse, die eine erheblich kostengünstigere Herstellung von Wasserstoff ermöglicht als dies mit dynamischen Elektrolyseprozessen zum Beispiel dem PEM-Verfahren möglich ist.

Erfindungsgemäß wird zur Herstellung des elektrischen Stroms für den Elektrolyseprozess eine Kombination von einem Verbrennungsprozess und einem Photovoltaikprozess eingesetzt. Wesentlich hierbei ist, dass zur Erzeugung des elektrischen Stroms nur regenerative Energiequellen eingesetzt werden, so dass eine umweltfreundliche Herstellung von Wasserstoff ermöglicht wird. Dabei liegt der Erfindung die Erkenntnis zugrunde, dass mit Photovoltaikanlage unter Ausnutzung der Sonne als regenerativer Energiequelle besonders umweltfreundlich und gleichzeitig auch wirtschaftlich, dass heißt mit geringen Kosten, elektrischer Strom erzeugt werden kann.

Der Nachteil von Photovoltaikanlagen, dass diese nicht fortdauernd elektrischen Strom erzeugen können, wird erfindungsgemäß dadurch kompensiert, dass der Photovoltaikprozess mit einem Verbrennungsprozess kombiniert wird, Wesentlich hierbei ist, dass zur Erzeugung des elektrischen Stroms nur regenerative Energiequellen beziehungsweise Energieträger eingesetzt werden, so dass eine umweltfreundliche Herstellung von Wasserstoff ermöglicht wird. Mit dem Verbrennungsprozess können dann die beim Photovoltaikprozess entstandenen Versorgungslücken geschlossen werden, dass heißt der Verbrennungsprozess wird insbesondere dann ergänzend zum Photovoltaikprozess herangeherangezogen, wenn die Photovoltaikanlage keinen elektrischen Strom produzieren kann oder nur unzureichend Strom produziert.

Besonders vorteilhaft wird als regenerativer Energieträger bei dem Verbrennungsprozess Biogas verwendet. Hierbei besteht ein wesentlicher Vorteil, dass bei Vergärungsprozessen gewonnenes Biogas wenigstens in gewissem Umfang in Gasbehälter gespeichert und damit gepuffert werden kann, bevor es insbesondere in Wärmekraftmaschinen verbrannt wird. Damit können mit diesem Verbrennungsprozess besonders flexibel Versorgungslücken, die bei dem Photovoltaikprozess systemimmanent auftreten, geschlossen werden.

Alternativ kann auch Biomasse als regenerativer Energieträger in einem Verbrennungsprozess zur Erzeugung von elektrischem Strom verbrannt werden.

Schließlich kann auch mit einer Abfallverbrennung elektrischer Strom erzeugt werden. Hier ist, um eine umweltverträgliche Herstellung des Wasserstoffs zu gewährleisten, einen möglichst aus regenerativen Stoffen bestehender Abfall zu wählen. Ein Beispiel hierfür ist die Verbrennung von Autoreifen.

Erfindungsgemäß wird die Erzeugung von elektrischem Strom mit einer Steuereinheit gesteuert.

Dabei wird mit der Steuereinheit der Zeitablauf vollständig gesteuert, zu welchen Zeiten und in welchem Umfang einerseits der Verbrennungsprozess und andererseits der Photovoltaikprozess zur Erzeugung des für den Elektrolyseprozess erforderlichen Stroms herangezogen wird.

Gemäß einer ersten Variante erfolgt die mit der Steuereinheit durchgeführte Steuerung derart, dass der elektrische Strom entweder aus einem Verbrennungsprozess oder aus einem Photovoltaikprozess erzeugt wird.

Alternativ erfolgt die mit der Steuereinheit durchgeführte Steuerung derart, dass der elektrische Strom aus vorgebbaren Anteilen sowohl aus einem Verbrennungsprozess als auch aus einem Photovoltaikprozess erzeugt wird.

Die so ausgebildete Steuereinheit arbeitet vorzugsweise völlig selbsttätig. Dabei kann zweckmäßig durch eine geeignete Ablaufsteuerung des Verbrennungsprozesses und des Photovoltaikprozesses eine Optimierung hinsichtlich der Verfügbarkeitszeiten der Photovoltaikanlage erfolgen, dass heißt es wird selbsttätig in der Steuereinheit ermittelt, zu welchen Zeiten mit dem Photovoltaikprozess genügend elektrischer Strom für den Elektrolyseprozess erzeugt wird und dann, wenn mit dem Photovoltaikprozess nur unzureichend elektrischer Strom erzeugt wird, der fehlende elektrische Strom mit einem Verbrennungsprozess erzeugt wird.

Ein weiterer wesentlicher Aspekt der Erfindung besteht darin, die Speicher zur Speicherung des Wasserstoffs in enger räumlicher Zuordnung zu den Anlagen zur Erzeugung des elektrischen Stroms und/oder zur Durchführung des Elektrolyseprozesses vorzusehen. Damit werden aufwändige Transporte des erzeugten Wasserstoffs zu Speichern an Verkaufsstätten, Tankstellen und dergleichen reduziert, was die Wirtschaftlichkeit des erfindungsgemäßen Verfahrens weiter erhöht.

Die Erfindung wird im Folgenden anhand der Zeichnung erläutert. Es zeigt:

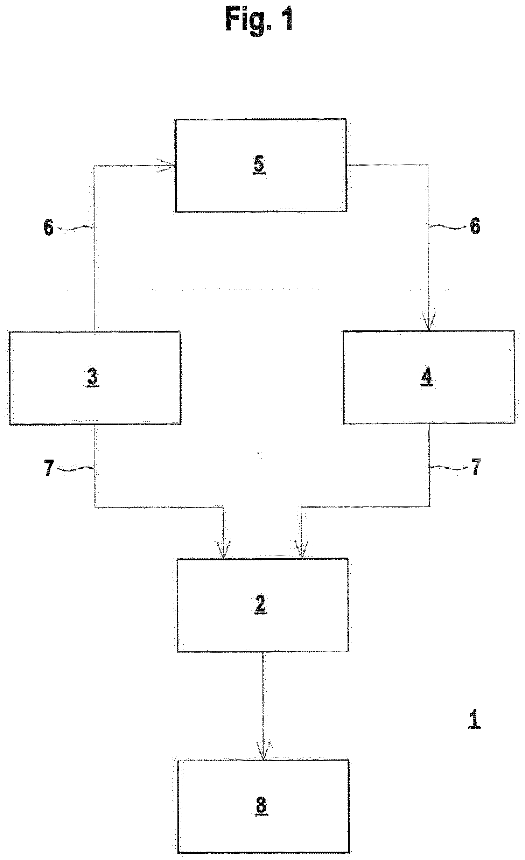

- Figur 1:: Ausführungsbeispiel einer Anlage zur Durchführung des erfindungsgemäßen Verfahrens.

Figur 1 zeigt ein Ausführungsbeispiel einer Anlage 1, mittels derer gemäß dem erfindungsgemäßen Verfahren Wasserstoff hergestellt wird.

Die Erzeugung von Wasserstoff erfolgt in einer Elektrolyseeinheit 2. Die Elektrolyseeinheit 2 ist so ausgebildet, dass in dieser ein kontinuierlich ablaufender Elektrolyseprozess erfolgt, mittels dessen Wasserstoff erzeugt wird. Im vorliegenden Fall erfolgt die Herstellung des Wasserstoffs mit einer alkalischen Elektrolyse.

Die Erzeugung des für die Elektrolyse erforderlichen elektrischen Stroms erfolgt möglichst ausschließlich unter Ausnutzung regenerativer Energiequellen beziehungsweise Energieträger. Erfindungsgemäß ist zur Erzeugung des elektrischen Stroms eine Verbrennungsanlage 3 und eine Photovoltaikanlage 4 vorgesehen. Prinzipiell ist auch eine Mehrfachanordnung von Verbrennungsanlagen 3 beziehungsweise Photovoltaikanlagen 4 möglich.

Im vorliegenden Fall wird mit der Verbrennungsanlage 3 Biogas als regenerativer Energieträger verbrannt. Das bei Vergärungsprozessen gewonnene Biogas wird in Behältern aufgefangen und von dort Wärmekraftmaschinen zugeführt, in welchen unter Verbrennung des Biogases elektrischer Strom erzeugt wird.

Prinzipiell kann auch eine Verbrennungsanlage 3 vorgesehen, in der Biomasse verbrannt wird. Schließlich ist auch eine geeignete Abfallverbrennung möglich.

Der Betrieb der Verbrennungsanlage 3 und Photovoltaikanlage 4 wird über eine Steuereinheit 5 gesteuert. Hierzu ist ein Rechnernetzwerk vorgesehen, das die Steuereinheit 5 über Zuleitungen 6 mit in der Verbrennungsanlage 3 beziehungsweise in der Photovoltaikanlage 4 vorgesehenen, nicht gesondert dargestellten Ablaufsteuerungen verbindet.

Die mit der Steuereinheit 5 durchgeführte Steuerung erfolgt allgemein derart, dass mit der Verbrennungsanlage 3 und der Photovoltaikanlage 4 kontinuierlich elektrischer Strom für die Elektrolyseeinheit 2 erzeugt wird, so dass dort der Elektrolyseprozess kontinuierlich ablaufen kann. Die Zuführung von elektrischem Strom von der Verbrennungsanlage 3 beziehungsweise der Photovoltaikanlage 4 erfolgt über geeignete Leitungen 7.

Dabei ist die Steuerung generell so angelegt, dass in den Zeiten, in denen die Photovoltaikanlage 4 keinen oder nur wenig elektrischen Strom produzieren kann, insbesondere bei Nacht oder starker Bewölkung, über die Steuereinheit 5 die Verbrennungsanlage 3 aktiviert wird, so dass diese den fehlenden Strom produziert.

Der in der Elektrolyseeinheit 2 erzeugte Wasserstoff wird in denen Speicher 8 gespeichert. Generell kann auch eine Mehrfachanordnung von Speichern 8 vorgesehen sein.

Vorteilhaft befindet sich der oder jeder Speicher 8 in unmittelbarer Nähe der Elektrolyseeinheit 2, so dass auch aufwändige Transportmittel zur Überführung des Wasserstoffs in den Speicher 8 verzichtet werden kann.

### Bezugszeichenliste

- (1): Anlage
- (2): Elektrolyseeinheit
- (3): Verbrennungsanlage
- (4): Photovoltaikanlage
- (5): Steuereinheit
- (6): Zuleitungen
- (7): Leitungen
- (8): Speicher

## Patentansprüche

1. Verfahren zur Erzeugung von Wasserstoff, **gekennzeichnet durch** folgende Verfahrensschritte: Erzeugung von elektrischem Strom aus einer Kombination eines Verbrennungsprozesses in Form einer Verbrennung von regenerativen Energieträgern und eines Photovoltaikprozesses, Nutzung des so erzeugten elektrischen Stroms für einen kontinuierlichen Elektrolyseprozess zur Erzeugung von Wasserstoff, wobei mit einer Steuereinheit (5) der Zeitablauf der Erzeugung von Strom vollständig gesteuert wird, wobei **durch** die Steuerung vorgegeben wird, zu welchen Zeiten und in welchem Umfang einerseits der Verbrennungsprozess und andererseits der Photovoltaikprozess zur Erzeugung des für den Elektrolyseprozess erforderlichen Stroms herangezogen wird.

2. Verfahren nach Anspruch 1, **dadurch gekennzeichnet, dass** der Elektrolyseprozess eine alkalische Elektrolyse ist.

3. Verfahren nach einem der Ansprüche 1 oder 2, **dadurch gekennzeichnet, dass** als regenerativer Energieträger Biogas eingesetzt wird.

4. Verfahren nach einem der Ansprüche 1 bis 3, **dadurch gekennzeichnet, dass** als regenerativer Energieträger Biomasse eingesetzt wird.

5. Verfahren nach einem der Ansprüche 1 bis 4, **dadurch gekennzeichnet, dass** ein Abfallverbrennungsprozess vorgesehen ist.

6. Verfahren nach einem der Ansprüche 1 bis 5, **dadurch gekennzeichnet, dass** der erzeugte Wasserstoff in wenigstens einem Speicher (8) gespeichert wird.

## Claims

1. Method of producing hydrogen, **characterised by** the following method steps:
generating electric current from a combination of a combustion process in the form of combustion of regenerative energy carriers and a photovoltaic process and using the thus-generated electric current for a continuous electrolysis process for producing hydrogen, wherein the time sequence of the generation of current is fully controlled by a control unit (5) and wherein it is predetermined by the control at which times and to what extent the combustion process on the one hand and the photovoltaic process on the other hand are utilised for generation of the current required for the electrolysis process.

2. Method according to claim 1, **characterised in that** the electrolysis process is an alkaline process.

3. Method according to claim 1 or 2, **characterised in that** biogas is used as regenerative energy carrier.

4. Method according to any one of claims 1 to 3, **characterised in that** biomass is used as regenerative energy carrier.

5. Method according to any one of claims 1 to 4, **characterised in that** a waste incineration process is provided.

6. Method according to any one of claims 1 to 5, **characterised in that** the hydrogen which is produced is stored in at least one store (8).

## Revendications

1. Procédé de production d'hydrogène, **caractérisé par** les étapes suivantes :
production de courant électrique à partir d'une combinaison d'un processus d'incinération sous la forme d'une combustion de sources d'énergie renouvelables et d'un processus photovoltaïque, utilisation du courant électrique ainsi produit pour un processus d'électrolyse continu pour produire de l'hydrogène, le déroulement dans le temps de la production de courant étant entièrement commandé par une unité de commande (5), la commande prescrivant à quels moments et dans quelle mesure d'une part le processus d'incinération et d'autre part le processus photovoltaïque sont utilisés pour produire le courant nécessaire pour le processus d'électrolyse.

2. Procédé selon la revendication 1, **caractérisé en ce que** le processus d'électrolyse est une électrolyse alcaline.

3. Procédé selon l'une des revendications 1 ou 2, **caractérisé en ce que** du biogaz est utilisé comme source d'énergie renouvelable.

4. Procédé selon l'une des revendications 1 à 3, **caractérisé en ce que** de la biomasse est utilisée comme source d'énergie renouvelable.

5. Procédé selon l'une des revendications 1 à 4, **caractérisé en ce qu'**un processus d'incinération de déchets est prévu.

6. Procédé selon l'une des revendications 1 à 5, **caractérisé en ce que** l'hydrogène produit est stocké dans au moins un réservoir (8).
